# EUROPEAN PATENT SPECIFICATION

(11) **EP 3 641 802 B1**
(45) Date of publication and mention of the grant of the patent: **13.05.2026**
(21) Application number: 17817070.0
(22) Date of filing: 22.06.2017
(51) Int. Cl.: C07K 16/28, A61K 39/00

(54) **METHODS AND PHARMACEUTICAL COMPOSITIONS FOR THE TREATMENT OF FIBROSIS WITH AGENTS CAPABLE OF INHIBITING THE ACTIVATION OF MUCOSAL-ASSOCIATED INVARIANT T (MAIT) CELLS**
VERFAHREN UND PHARMAZEUTISCHE ZUSAMMENSETZUNGEN ZUR BEHANDLUNG VON FIBROSE MIT MITTELN ZUR HEMMUNG DER AKTIVIERUNG VON MUKOSA-ASSOZIIERTEN INVARIANTEN T-ZELLEN (MAIT)
MÉTHODES ET COMPOSITIONS PHARMACEUTIQUES POUR LE TRAITEMENT DE LA FIBROSE AVEC DES AGENTS CAPABLES D'INHIBER L'ACTIVATION DE CELLULES T INVARIANTES ASSOCIÉES AUX MUQUEUSES (MAIT)

(43) Date of publication of application: 29.04.2020
(73) Proprietor: INSERM (Institut National de la Santé et de la Recherche Médicale), 75013 Paris (FR); Université Paris Cité, 75006 Paris (FR); Centre National de la Recherche Scientifique (CNRS), 75016 Paris (FR); Assistance Publique - Hôpitaux de Paris, 75004 Paris (FR)
(72) Inventor: LOTERSZTAJN, Sophie, 75870 Paris (FR); WAN, JingHong, 75870 Paris Cedex 18 (FR); PARADIS, Valérie, 92110 Clichy (FR); LEHUEN, Agnès, 75014 Paris (FR); HEGDE, Pushpa, 75870 Paris Cedex 18 (FR); WEISS, Emmanuel, 75018 Paris (FR)
(74) Representative: Inserm Transfert
(86) International application number: PCT/IB2017/001326
(87) International publication number: WO 2018/234843

(56) References cited:
- WO-A1-2014/005194
- PUSHPA HEGDE, EMMANUEL WEISS, GLADYS FERRERE, ABHISHAK GUPTA, BADR KIAF, JUNGHONG WAN, VALERIE PARADIS, RICHARD MOREAU ET AL.: "189: Profibrogenic functions of mucosal-associated invariant T (MAIT) cells during chronic liver injury", HEPATOLOGY, vol. 64, no. 1, supplement 1, 189, 11 November 2016 (2016-11-11) - 15 November 2016 (2016-11-15), pages 99A - 100A, XP009555792, ISSN: 1527-3350, DOI: 10.1002/hep.28796
- JEFFERY HANNAH C ET AL: "Biliary epithelium and liver B cells exposed to bacteria activate intrahepatic MAIT cells through MR1", JOURNAL OF HEPATOLOGY, ELSEVIER, AMSTERDAM, NL, vol. 64, no. 5, 29 December 2015 (2015-12-29), pages 1118 - 1127, XP029506666, ISSN: 0168-8278, DOI: 10.1016/J.JHEP.2015.12.017
- MABIRE MORGANE ET AL: "MAIT cell inhibition promotes liver fibrosis regression via macrophage phenotype reprogramming", NATURE COMMUNICATIONS, vol. 14, no. 1, 1 April 2023 (2023-04-01), UK, XP093362586, ISSN: 2041-1723, Retrieved from the Internet <URL:https://www.nature.com/articles/s41467-023-37453-5> DOI: 10.1038/s41467-023-37453-5
- AYAKO KURIOKA ET AL: "MAIT cells: new guardians of the liver", CLINICAL & TRANSLATIONAL IMMUNOLOGY, vol. 5, no. 8, 19 August 2016 (2016-08-19), pages e98, XP055441541, DOI: 10.1038/cti.2016.51
- TOUBAL AMINE ET AL: "Lights on MAIT cells, a new immune player in liver diseases", JOURNAL OF HEPATOLOGY, ELSEVIER, AMSTERDAM, NL, vol. 64, no. 5, 8 February 2016 (2016-02-08), pages 1008 - 1010, XP029506682, ISSN: 0168-8278, DOI: 10.1016/J.JHEP.2016.02.003

## Description

### FIELD OF THE INVENTION:

The present invention relates to methods and pharmaceutical compositions for the treatment of fibrosis with agents capable of inhibiting the activation of mucosal-associated invariant T (MAIT) cells.

### BACKGROUND OF THE INVENTION:

Hepatic fibrosis, the common response to chronic liver injury, ultimately leads to cirrhosis, a major public health problem worldwide ^{1,2}. In western countries, the prevailing causes of fibrosis and cirrhosis include chronic alcohol consumption and non-alcoholic fatty liver disease associated with obesity and type-2 diabetes ⁴. Cirrhosis lacks definitive treatment, and liver transplantation is considered as the only option for end-stage liver disease. Recent advances in the understanding of liver fibrosis pathogenesis have revealed that sustained inflammation originating from resident and infiltrating immune cells drives the fibrogenic process via direct effects on fibrogenic cell proinflammatory and profibrogenic functions, and also contributes to its regression ^{1,2}. In recent years, monocytes/macrophages have received the most interest, but much less is known about the contribution and functions of T cell subsets in the fibrogenic process, in particular regarding the possible impact of innate-like lymphoid cells.

Mucosal-associated invariant T (MAIT) cells are non-conventional T cells that express an evolutionarily conserved semi-invariant T cell antigen receptor (TCR) repertoire (made of an invariant Vα7.2-Jα33 in humans and Vα19-Jα33 in mice) and are restricted by the non-classical MHC-related molecule 1 (MR1) ³. They are abundant in human blood, gut and liver, and secrete cytokines such as IL-17, granzyme B, IFN-γ and TNF-α. In healthy individuals, MAIT cells play a defensive role against pathogens, by protecting epithelial and mucosal layer integrity against bacterial invasion and viral infections, in particular in the liver ^{3,5,6,7}. However, their role in inflammatory diseases has only recently emerged ⁸, with consistent data reporting altered MAIT cells functions during acute and chronic inflammatory injury ⁹⁻¹¹.

Kurioka, Ayako et al. explores the role of MAIT cells in human liver and their involvement in various liver diseases (Kurioka, Ayako et al. "MAIT cells: new guardians of the liver." Clinical & translational immunology vol. 5,8 e98. 19 Aug. 2016).

Toubal, Amine, and Agnès Lehuen reviews the potential role of MAIT cells in diseases, in particular in liver diseases (Toubal, Amine, and Agnès Lehuen. "Lights on MAIT cells, a new immune player in liver diseases." Journal of hepatology vol. 64,5 (2016): 1008-1010).

WO2014/005194 discloses ligands which binds to Major histocompatibility complex-related protein 1 (MR1), wherein said binding results in binding of the MR1 to MAIT cells.

Pushpa Hedge et al. discloses that chronic liver injury is associated with dysregulation of MAIT cell functions (Pushpa Hedge et al. : 189: Profibrogenic functions of mucosal-associated invariant T (MAIT) cells during chronic liver injury", Hepatology, vol.64, no. 1, supplement 1, 189, 11 November 2016, pages 99A-100A).

### SUMMARY OF THE INVENTION:

The present invention relates to an agent capable of inhibiting the activation of Mucosal-Associated Invariant T (MAIT) cells for use in a curative method of treating liver fibrosis in a patient in need thereof, wherein the agent is:
- An anti-MHC-related molecule 1 (MR1) neutralizing antibody or
- A small organic molecule selected from the group consisting of 6-formyl pterin and acetyl-6-formylpterin (Ac-6-FP).

In particular, the present invention is defined by the claims.
The references to methods of treatment in this description are to be interpreted as references to the compounds, pharmaceutical compositions and medicaments of the present invention for use in a method for the treatment of the human body by therapy.

### DETAILED DESCRIPTION OF THE INVENTION:

Persistent inflammation is a driving force of liver fibrosis progression. Mucosal-Associated Invariant T (MAIT) cells are non-conventional T cells that display altered functions during chronic inflammatory diseases. Here, the inventors hypothesized that MAIT cells may contribute to the fibrogenic process. They report a loss of circulating MAIT cells in cirrhotic patients and their hepatic accumulation in an activated phenotype within the fibrotic septa. Using two models of chronic liver injury, the inventors demonstrate that mice enriched in MAIT cells (Vα19TCRTg) show exacerbated liver fibrosis and higher number of hepatic fibrogenic cells than wild type counterparts, whereas MAIT cell-deficient mice (MR1^{-/-}mice) are resistant. Co-culture experiments indicate that profibrogenic properties of activated human MAIT cells are related to both mitogenic effects on human hepatic myofibroblasts, in an MR1-dependent manner, and pro-inflammatory features, as a result of TNF-α production. The results highlight the profibrogenic functions of MAIT cells and suggest that targeting MAIT cells may constitute an attractive antifibrogenic strategy during chronic liver injury.

Accordingly, the first object of the present invention relates to an agent capable of inhibiting the activation of Mucosal-Associated Invariant T (MAIT) cells for use in a curative method of treating liver fibrosis in a patient in need thereof wherein the agent is:
- An anti-MHC-related molecule 1 (MR1) neutralizing antibody or
- A small organic molecule selected from the group consisting of 6-formyl pterin and acetyl-6-formylpterin (Ac-6-FP).

As used herein, the term "treatment" or "treat" refer to curative or disease modifying treatment, including treatment of patients who are ill or have been diagnosed as suffering from a disease or medical condition, and includes suppression of clinical relapse. The treatment may be administered to a subject having a medical disorder, in order to cure, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

As used herein, the term "fibrosis" refers to the formation of fibrous tissue as a reparative or reactive process, rather than as a normal constituent of an organ or tissue. Fibrosis is characterized by myofibroblast accumulation and collagen deposition in excess of normal deposition in any particular tissue. The term is used synonymously with "myofibroblast accumulation and collagen deposition". The fibrosis affects at least one organ selected from the group consisting of skin, eye, intestine, heart, liver, lung, and kidney. Examples of fibrosis include, without limitation, dermal scar formation, keloids, liver fibrosis, lung fibrosis, kidney fibrosis, glomerulosclerosis, pulmonary fibrosis (e.g. idiopathic pulmonary fibrosis), liver fibrosis (e.g. following liver transplantation, liver fibrosis following chronic hepatitis C virus infection), renal fibrosis, intestinal fibrosis, interstitial fibrosis, cystic fibrosis of the pancreas and lungs, injection fibrosis, endomyocardial fibrosis, mediastinal fibrosis, myelofibrosis, retroperitoneal fibrosis, progressive massive fibrosis, nephrogenic systemic fibrosis... The fibrosis may be caused by surgical implantation of an artificial organ.

In the context of the invention, the fibrosis occurs in the liver (i.e. "liver fibrosis" or "hepatic fibrosis") as a part of the wound- healing response to chronic liver injury. Liver fibrosis is characterized by the accumulation of extracellular matrix that can be distinguished qualitatively from that in normal liver. Left unchecked, hepatic fibrosis progresses to cirrhosis (defined by the presence of encapsulated nodules), liver and organ failure, and death. Chronic liver injury may be the result of chronic alcohol consumption (alcoholic liver disease, steatohepatitis (ASH)), overfeeding, insulin resistance, type 2 diabetes (non-alcoholic fatty liver disease, NASH, steatosis), idiopathic portal hypertension, hepatic fibrosis (including congenital hepatic fibrosis), autoimmune hepatitis, primary sclerosing cholangitis, or primary biliary cirrhosis. In some embodiments, the fibrosis is associated with liver steatosis.

In some embodiments, the fibrosis is the result of injury, surgery, or radiation. As used herein, the term "MAIT cells" or "Mucosal-Associated Invariant T cells" refers to a population of T cells present in mammals, preferably humans, that display an invariant TCR alpha chain comprising Vα7.2-Jα33 (in humans), a CDR3 of constant length, and a limited number of Vβ segments together with an activated phenotype (CD44) (see, e.g., Lantz and Bendelac. 1994. J. Exp Med. 180:1097-106; Tilloy et al., J. Exp. Med., 1999, 1907-1921; Treiner et al. (2003) Nature 422 164-169). MAIT cells are generally CD8⁺ (expressing mostly the homodimeric form of CD8αα) or CD4⁻/CD8⁻ (DN), and are restricted by the non-classical MHC class I molecule MR1. For the purposes of the present invention, any T cells that express the invariant Vα7.2-Jα33 alpha TCR chain are considered to be MAIT cells. Typically, the alpha chain is associated with an invariant CDR3 and with either Vβ2 or Vβ13.

As used herein, the expression "agent capable of inhibiting the activation of MAIT cells" refers to any refers to any molecule that under cellular and/or physiological conditions is capable of inhibiting the profibrogenic functions of MAIT cells.

Inhibitors of MAIT cells are known in the art and typically include those described in Corbett, A.J. et al. T-cell activation by transitory neo-antigens derived from distinct microbial pathways. Nature 509, 361-365 (2014); and Keller AN et al. Drugs and drug-like molecules can modulate the function of mucosal-associated invariant T cells Nat Immunol. 2017 Apr;18(4):402-411. Other examples include those described in the International Patent Application WO 2014005194. In some embodiments, the inhibitor is selected from the group consisting of 6-formyl pterin and acetyl-6-formylpterin (Ac-6-FP).

In some embodiments, the agent is an MR1 neutralizing antibody. As used herein, the term "antibody" is thus used to refer to any antibody-like molecule that has an antigen binding region, and this term includes antibody fragments that comprise an antigen binding domain such as Fab', Fab, F(ab')2, single domain antibodies (DABs), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lamda) bodies (scFv-CL fusions); BiTE (Bispecific T-cell Engager, scFv-scFv tandems to attract T cells); DVD-Ig (dual variable domain antibody, bispecific format); SIP (small immunoprotein, a kind of minibody); SMIP ("small modular immunopharmaceutical" scFv-Fc dimer; DART (ds-stabilized diabody "Dual Affinity ReTargeting"); small antibody mimetics comprising one or more CDRs and the like. The techniques for preparing and using various antibody-based constructs and fragments are well known in the art (see Kabat et al., 1991). Diabodies, in particular, are further described in EP 404, 097 and WO 93/1 1 161 ; whereas linear antibodies are further described in Zapata et al. (1995). Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, Fv, dsFv, Fd, dAbs, TandAbs, ds-scFv, dimers, minibodies, diabodies, bispecific antibody fragments and other fragments can also be synthesized by recombinant techniques or can be chemically synthesized. Techniques for producing antibody fragments are well known and described in the art. For example, each of Beckman et al., 2006; Holliger & Hudson, 2005; Le Gall et al., 2004; Reff & Heard, 2001 ; Reiter et al., 1996; and Young et al., 1995 further describe and enable the production of effective antibody fragments. In some embodiments, the antibody of the present invention is a single chain antibody. As used herein the term "single domain antibody" has its general meaning in the art and refers to the single heavy chain variable domain of antibodies of the type that can be found in Camelid mammals which are naturally devoid of light chains. Such single domain antibody are also "nanobody^{®}". For a general description of (single) domain antibodies, reference is also made to the prior art cited above, as well as to EP 0 368 684, Ward et al. (Nature 1989 Oct 12; 341 (6242): 544-6), Holt et al., Trends Biotechnol., 2003, 21(11):484-490; and WO 06/030220, WO 06/003388.

As used herein the term "bind" indicates that the antibody has affinity for the surface molecule. The term "affinity", as used herein, means the strength of the binding of an antibody to an epitope. The affinity of an antibody is given by the dissociation constant Kd, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Ka is defined by 1/Kd. Preferred methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983). One preferred and standard method well known in the art for determining the affinity of mAbs is the use of Biacore instruments.

In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CHI, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) can participate to the antibody binding site or influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L- CDR3 and H-CDR1, H-CDR2, H-CDR3, respectively. An antigen-binding site, therefore, typically includes six CDRs, comprising the CDR set from each of a heavy and a light chain V region. Framework Regions (FRs) refer to amino acid sequences interposed between CDRs. The residues in antibody variable domains are conventionally numbered according to a system devised by Kabat et al. This system is set forth in Kabat et al., 1987, in Sequences of Proteins of Immunological Interest, US Department of Health and Human Services, NIH, USA (hereafter "Kabat et al."). This numbering system is used in the present specification. The Kabat residue designations do not always correspond directly with the linear numbering of the amino acid residues in SEQ ID sequences. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35B (H-CDR1), residues 50-65 (H-CDR2) and residues 95-102 (H-CDR3) according to the Kabat numbering system. The CDRs of the light chain variable domain are located at residues 24-34 (L-CDR1), residues 50-56 (L-CDR2) and residues 89-97 (L-CDR3) according to the Kabat numbering system.

In some embodiments, the antibody is a humanized antibody. As used herein, "humanized" describes antibodies wherein some, most or all of the amino acids outside the CDR regions are replaced with corresponding amino acids derived from human immunoglobulin molecules. Methods of humanization include, but are not limited to, those described in U.S. Pat. Nos. 4,816,567, 5,225,539, 5,585,089, 5,693,761, 5,693,762 and 5,85 9,205.

In some embodiments, the antibody is a fully human antibody. Fully human monoclonal antibodies also can be prepared by immunizing mice transgenic for large portions of human immunoglobulin heavy and light chain loci. See, e.g., U.S. Pat. Nos. 5,591,669, 5,598,369, 5,545,806, 5,545,807, 6,150,584, and references cited therein.

In some embodiments, the agent is an anti-MR1 neutralizing antibody that block the presentation of antigenic ligands (e.g. microbial vitamin B metabolites) by MR1. In some embodiments, the MR1 neutralizing antibodies block the interaction between MR1 the Vα7.2-Jα33 receptors. These antibodies are thus referred to as "neutralizing" or "inhibitory" or "blocking" antibodies. In some embodiments, the agent is an anti-MR1 neutralizing antibody. Such antibodies are useful, inter alia, for decreasing MAIT immune cell activity.

In some embodiments, the neutralizing antibodies of the present invention does not mediate antibody-dependent cell-mediated cytotoxicity and thus does not comprise an Fc portion that induces antibody dependent cellular cytotoxicity (ADCC). In some embodiments, the neutralizing antibody does not comprise an Fc domain capable of substantially binding to a FcgRIIIA (CD16) polypeptide. In some embodiments, the neutralizing antibody lacks an Fc domain (e.g. lacks a CH2 and/or CH3 domain) or comprises an Fc domain of IgG2 or IgG4 isotype. In some embodiments, the neutralizing antibody consists of or comprises a Fab, Fab', Fab'-SH, F (ab') 2, Fv, a diabody, single-chain antibody fragment, or a multispecific antibody comprising multiple different antibody fragments. In some embodiments, the neutralizing antibody is not linked to a toxic moiety. In some embodiments, one or more amino acids selected from amino acid residues can be replaced with a different amino acid residue such that the antibody has altered C2q binding and/or reduced or abolished complement dependent cytotoxicity (CDC). This approach is described in further detail in U.S. Patent Nos. 6,194,551 by ldusogie et al.

A "therapeutically effective amount" refers to an amount effective of the agent, at dosages and for periods of time necessary, to achieve a desired therapeutic result. A therapeutically effective amount of drug may vary according to factors such as the disease state, age, sex, and weight of the individual, and the ability of drug to elicit a desired response in the individual. A therapeutically effective amount is also one in which any toxic or detrimental effects of the antibody or antibody portion are outweighed by the therapeutically beneficial effects. The efficient dosages and dosage regimens for drug depend on the disease or condition to be treated and may be determined by the persons skilled in the art. A physician having ordinary skill in the art may readily determine and prescribe the effective amount of the pharmaceutical composition required. For example, the physician could start doses of drug employed in the pharmaceutical composition at levels lower than that required in order to achieve the desired therapeutic effect and gradually increase the dosage until the desired effect is achieved. In general, a suitable dose of a composition of the present invention will be that amount of the compound which is the lowest dose effective to produce a therapeutic effect according to a particular dosage regimen. Such an effective dose will generally depend upon the factors described above.

Typically, the agent of the present invention is administered to the subject in the form of a pharmaceutical composition which comprises a pharmaceutically acceptable carrier. Pharmaceutically acceptable carriers that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat. For use in administration to a subject, the composition will be formulated for administration to the subject. The compositions of the present invention may be administered orally, parenterally, by inhalation spray, topically, rectally, nasally, buccally, vaginally or via an implanted reservoir. The used herein includes subcutaneous, intravenous, intramuscular, intra-articular, intra-synovial, intrasternal, intrathecal, intrahepatic, intralesional and intracranial injection or infusion techniques. Sterile injectable forms of the compositions of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. The compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include, e.g., lactose. When aqueous suspensions are required for oral use, the active ingredient is combined with emulsifying and suspending agents. If desired, certain sweetening, flavoring or coloring agents may also be added. Alternatively, the compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols. The compositions of this invention may also be administered topically, especially when the target of treatment includes areas or organs readily accessible by topical application, including diseases of the eye, the skin, or the lower intestinal tract. Suitable topical formulations are readily prepared for each of these areas or organs. For topical applications, the compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water. Topical application for the lower intestinal tract can be effected in a rectal suppository formulation (see above) or in a suitable enema formulation. Patches may also be used. The compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. For example, an antibody present in a pharmaceutical composition of this invention can be supplied at a concentration of 10 mg/mL in either 100 mg (10 mL) or 500 mg (50 mL) single-use vials. The product is formulated for IV administration in 9.0 mg/mL sodium chloride, 7.35 mg/mL sodium citrate dihydrate, 0.7 mg/mL polysorbate 80, and Sterile Water for Injection. The pH is adjusted to 6.5. An exemplary suitable dosage range for an antibody in a pharmaceutical composition of this invention may between about 1 mg/m² and 500 mg/m². However, it will be appreciated that these schedules are exemplary and that an optimal schedule and regimen can be adapted taking into account the affinity and tolerability of the particular antibody in the pharmaceutical composition that must be determined in clinical trials. A pharmaceutical composition of the invention for injection (e.g., intramuscular, i.v.) could be prepared to contain sterile buffered water (e.g. 1 ml for intramuscular), and between about 1 ng to about 100 mg, e.g. about 50 ng to about 30 mg or more preferably, about 5 mg to about 25 mg, of the inhibitor of the invention.

The invention will be further illustrated by the following figures and examples. However, these examples and figures should not be interpreted in any way as limiting the scope of the present invention.

### FIGURES:

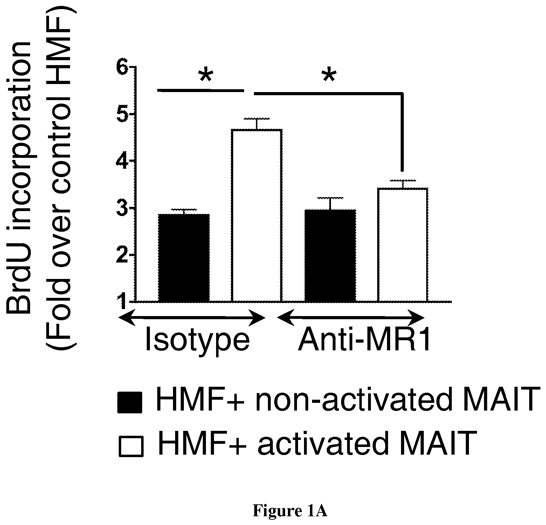

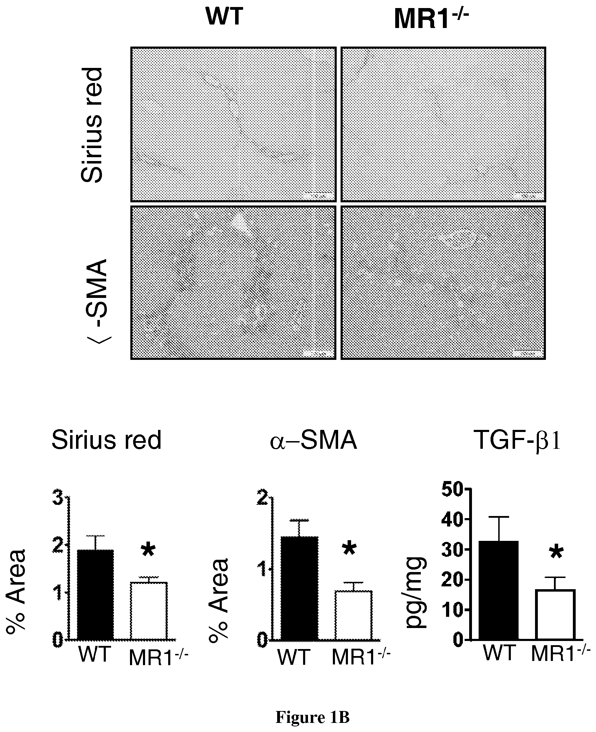

**Figure 1****: Inhibition of MAIT-cell induced profibrogenic effect *in vitro* by neutralising MR1 and *in vivo* in MR1 deficient mice.** (A) DNA synthesis in hepatic myofibroblasts pre-treated with MR1 neutralizing antibody or isotype, and co-cultured with either activated or non-activated MAIT cells. Results show a representative experiment and is the mean±SEM of quadruplicate determinations. Similar results were obtained in 2 independent experiments with MAIT cell from 2 different donors. (B) Representative images and quantification of Sirius red and *α* -SMA areas, and hepatic TGF-b1 secretion in MAIT cell-deficient (MR1^{-/-}) mice (n=6) and their WT littermates (n=5) chronically administered CCl₄.

### EXAMPLE:

### Material & Methods

### Human blood samples.

Blood samples were obtained with written informed consent from 39 patients with stable biopsy-proven cirrhosis, from alcoholic or non-alcoholic fatty liver disease-related cirrhosis hospitalized at Hopital Beaujon (Clichy, France) (Supplementary Table 1). The study was approved by the local ethics committees (Comité d'Evaluation de l'Ethique des projets de Recherche Biomédicale (CEERB) Paris Nord: n°16-039). Blood from healthy volunteers (n=29) was obtained through a formalized agreement with Etablissement Français du Sang (agreement n° (n°2015012778).

### Human liver samples

Non tumoral livers (n=7 and n=5 control and n=7 and n=5 cirrhotic patients for FACS analysis and immunohistochemistry, respectively) were obtained from surgical samples (resection or liver transplantation) at distance, when present, from tumor nodule. Liver tissue was processed as rapidly as possible after resection, frozen in liquid nitrogen, and stored at - 80°C (Biobank Pathology Dpt, Beaujon hospital, DC-2009-938). For all cases, fibrosis staging was assessed according to Metavir system ¹⁶. All patients signed an informed consent form and the study was approved by the local Ethics Committee.

### Peripheral blood mononuclear cells (PBMC) isolation.

PBMC were prepared from the blood using ficoll hypacque (GE Healthcare) density gradient centrifugation, as previously described ¹⁷, and freshly used for the analysis of surface phenotype of MAIT cells. For the detection of cytokine production, PBMC were stimulated for 6 hours at 37°C with PMA and ionomycin (Sigma-Aldrich) at 25 ng/ml and 1 µg/ml, respectively, in the presence of brefeldin A at 10 µg/ml (Biolegend) in RPMI medium supplemented with 10% fetal bovine serum (Life Technologies).

### Flow cytometric analysis

Flurochrome conjugated Anti-CD3, anti-CD4 (OKT4), anti-CD161(HP-3G10), anti-Vα7.2⁺(3C10) , anti-CD25 (BC96), anti-CD69 (FN50) anti-CCR6 (G034E3), and anti-Bcl-2 (clone 100) Anti-IFN-y (4S.B3), anti-Granzyme B (GB11), anti- TNF-α (MAb11), anti-IL-17 (BL168), anti-Ki-67 (B56) antibodies were obtained from Biolegend France. Anti-CD8α (SK1), anti-TCRγδ (B1) antibodies were obtained from BD Biosciences. Dead cells were excluded from the analysis using the fixable viability dye eflour 506 (eBiosciensces). MAIT cells were identified by multicolour flow cytometry as CD3⁺CD4⁻CD8⁺CD161^{hi}Vα7.2⁺ cells. Intracellular cytokines were analyzed using PMA/ionomycin/BrefeldinA treated PBMC using the intracellular cytokine staining kit (BD Biosciences) according to manufacture's instruction. Analysis of Ki-67 and bcl2 in PBMC were performed using the intracellular transcription factor staining kit (eBioscience). Data acquisition were performed using a BD Biosciences LSR Fortessa cytometer and data were analysed using FlowJo analysis software (Tree Star).

### Human MAIT cell isolation

MAIT cells were isolated from PBMC prepared from buffy coats of healthy donors, as previously described ⁹. Briefly, monocytes and CD4 T cells were sequentially depleted from PBMC by adhesion on polystyrene culture flasks for three hours at 37°C, and anti CD4 microbeads (Miltenyi Biotech France), respectively. Vα7.2⁺ cells were isolated from the non-monocyte and non-CD4 PBMC fraction, using an anti Vα7.2⁺ antibody conjugated to FITC, followed by a positive selection with anti-FITC microbeads (Miltenyi Biotech, France) and used as enriched MAIT cells for co-culture experiments. The purity of isolated Vα7.2⁺ cells was > 80% and > 95% of the isolated Vα7.2⁺ cells were co-expressing CD161. > 95% of the isolated CD161^{hi} Vα7.2⁺ cells were positive to APC conjugated 5-OP-RU loaded MR1 tetramers (¹⁸, NIH tetramer Core Facility, Emory University Vaccine Center, Atlanta, USA) (Extended data Figure 4a). MAIT cells added in co-culture experiments were either left non-activated or were activated using anti-CD3 (HIT3a) at 2.5µg/ml, soluble anti CD28 (CD28.2) (1g/µml) and IL-7 10ng/ml (Biolegend) ¹⁹.

### Co-culture of human MAIT cells with human hepatic myofibroblasts

Human hepatic myofibroblasts (HMF) were obtained by outgrowth of explants prepared from surgical specimen of normal human liver, as we previously described ²⁰. This procedure was performed in accordance with ethical regulations imposed by the French legislation. The fibrogenic phenotype of these cells has been extensively characterized ²⁰. Cells were grown to 80% confluency in RPMI containing 10% fetal calf serum and serum-deprived for two days before adding freshly isolated non-activated or activated MAIT cells, at the ratio of 1:10 (HMF: MAIT cells).

### DNA synthesis assay

BrdU (Roche, France) was added at the bottom of the well (transwell experiments), or to the co-culture medium for 18 hours. In co-culture experiments, MAIT cells were then carefully aspirated, and adherent HMF were washed once with 1X PBS and their DNA synthesis estimated by a colorimetric BrdU ELISA test (Roche, France) as per the manufacturer's instruction. For the experiments with MR1 blocking antibodies, human hepatic myofibroblasts were incubated with purified anti MR1 antibody at 20µg/ml (26.5, Biolegend, France) or with isotype control antibody for 2 hours at 37°C. Pre-activated or non-activated MAIT cell were washed and co-cultured with anti-MR1 (26.5, Biolegend )-exposed HMF. Cells were then processed for DNA synthesis as described above. In separate experiments, FACS analysis of Ki-67 was performed in HMF upon co-culture with MAIT cells, using phycoerythrin (PE) conjugated anti-Ki67 (Biolegend, France) by intracellular staining as per the manufacturer's instructions.

### Analysis of expression of MR1 on hepatic myofibroblasts by immunocytochemistry

HMF were fixed in 4% paraformaldyhyde, followed by incubation with a blocking buffer 1% BSA-PBS (0,1% Triton x100) and with anti-MR1 antibody (clone 26.5, mouse IgG2a, kappa, 1:25, Biolegend,) and Goat-anti- mouse IgG (H+L) secondary antibody, Alexa Fluor 555 (1: 1000, Invitrogen,). Nuclear staining was performed using Prolong Gold antifade mountant with DAPI (Invitrogen,). No staining was observed with the isotype (clone MOPC-173, mouse IgG2a, kappa, 1:25, Biolegend,). Cells were visualized using confocal microscopy (Confocal Zeiss LSM 780).

### Analysis of surface expression of MR1 on hepatic myofibroblasts by flow cytometry

Hepatic myofibroblast cultures or HMF/MAIT cell co-cultures were exposed to1µM Acetyl 6-formylpterin (Ac 6-FP) (Schircks Laboratories, Switzerland) for 2 hrs at 37°C. Cells were then washed, trypsinised, labeled with PE-conjugated anti MR1 antibody (26.5)/isotype control and subjected to flow cytometric analysis.

### Analysis of cytokine and chemokine production by hepatic myofibroblasts

Hepatic myofibroblasts were co-cultured with either non-activated or activated MAIT cells for 18 hours. Brefeldin A (10 µg/ml) was added for the last 3 hours, and cells were analysed for intracellular cytokines and chemokines, using the intracellular cytokine staining kit (BD Biosciences). When indicated, IL-17 neutralizing antibody (64CAP17, eBioscience) or isotype control antibody was added to the co-culture.

### Animals MR1^{-/-}

Animals MR1^{-/-} C57BL/6 ²⁷, Vα19 Tg C57BL/6 mice ⁴⁰ and their WT counterparts were generated as described in ^{27,40}. Since MR1 is the restriction molecule required for MAIT thymic development, MR1^{-/-} C57BL/6 do not have MAIT cells. On the contrary, mice expressing the Va19-Ja33 TCR transgene have a 10-fold increase in MAIT cell frequency in the various tissues such as spleen, liver, colon, lymph nodes.

### Mice models of liver fibrosis

Animals were housed in pathogen-free animal facility and fed ad libitum. Liver fibrosis was induced in male mice by either repeated injections of carbon tetrachloride (CCl₄, 0.5 ml/kg body weight, 1:10 dilution in mineral oil [MO; Sigma, France), twice a week for 4 weeks (Vα19 TCR transgenic, n=9; WT littermates n=10; MR1^{-/-}, n=6; WT littermates n=5), or bile duct ligation and section (Vα19 TCR transgenic, n=9; WT littermates n=3), as we previously described ^{21,22}. Animals were sacrificed 24 h after the last CCl₄ injection or 12 days after surgery in BDL mice. There was no difference in the frequency of B lymphocytes, neutrophils, macrophages and dendritic cells between the two groups in the different models. Moreover, the extent of injury was similar between groups in all the models, as reflected by similar increases in serum transaminases measurements (data not shown). Experiments were performed in accordance with protocols approved by the Paris-Nord ethical committee C2EA 121 (authorization number 02529.02).

### Histological analysis

Hematoxylin and eosin and Sirius Red staining were performed on 4-µm thick formalin-fixed paraffin-embedded tissue sections at the Pathology Department of Hopital Bichat, Paris, France. Sirius Red-stained areas from 10 fields (magnification x20) from each mouse were quantified with Image J.

### Immunohistochemistry

Mice liver. Immunohistochemical detection of ACTA2 was carried out as previously described ²¹ on paraffin-embedded mice liver tissue sections (4 µm) using the MOM immunodetection kit (Vector, PK2002) and a mouse monoclonal anti-ACTA2 antibody (1:1000, Sigma, 2547) according to manufacturer's instructions. ACTA2 positive area from ten fields (magnification x20) from 3-7 mice/group were quantified with ImageJ. No staining was observed when the primary antibody was omitted.

Human liver. Va7.2 immunodetection was performed in frozen sections of normal or subnormal liver samples (n=5) from patients that underwent resection surgeries for non- hepatocellular primary tumor (n=3) or colorectal cancer liver metastasis (n=2), and showed no (F0) or mild (F1) fibrosis and no alteration in liver biological tests. Liver samples from patients with cirrhosis, that were obtained from non-tumoral part of HCC resection (n=3) and liver explant during liver transplantation (n=2). Briefly, tissue sections were fixed in 4% paraformaldehyde for 15 min, and incubated overnight at 4 °C with purified anti-human Va7.2 antibody (1:50, #351702, Biolegend), following a blocking step (PBS containing 10% goat serum, 1 % BSA and 0.2% Triton X-100). After washes, sections were incubated with Alexa Fluor 488 goat anti-mouse IgG (1:200, A-11001, Life Technologies) for 1 h at room temperature. Nuclear counterstaining was obtained with DAPI (1:10000, #62248, Thermo). Va7.2-positive cells were semi-quantitatively assessed (0: absent; +:positive; ++ strongly positive) and their location determined (sinusoidal space and mesenchymal space).

### Statistical analysis

Results are expressed as mean ± standard error of the mean (SEM). Nonparametric tests were performed using Mann-Whitney U test or Student t test, as appropriate. All P values are 2-sided, and P values less than 0.05 were considered to be statistically significant. Analyses were performed using GraphPad Prism version 6.

### Results

### The frequency and functions of circulating MAIT cells are altered in cirrhotic patients

We evaluated the frequency of circulating T cell subsets in the peripheral blood mononuclear cells (PBMC) from cirrhotic patients with alcoholic and non-alcoholic fatty liver disease (n=39), and compared to that of healthy donors (n=29). There was a decrease in CD8⁺ positive cells and a slight, but significant increase in the CD4⁺ population in cirrhotic patients. Detailed analysis of innate-like T cell populations showed a small decrease in the frequency of iNKT cells in cirrhotic patients, and no change in γδT cells. However, as compared to healthy donors, the median MAIT cell frequency, identified as CD3⁺CD4⁻ CD161^{hi} Vα7.2⁺ cells within the CD3+ population, was strongly decreased in cirrhotic patients (3.010%±0.38 in healthy donors, within the range reported in other studies vs 0.39%±0.11 %, in cirrhotic patients). The majority of MAIT cells from healthy donors and cirrhotic patients were either CD8α⁺ (nearly 80 %) or double negative (up to 20%). Blood MAIT cells from cirrhotic patients displayed an activated phenotype, characterized by higher frequencies of CD25⁺ and CD69⁺ MAIT cells vs healthy donors, that were negatively correlated with MAIT cell frequency. They also produced more IL-17 and granzyme B than healthy MAIT cells, whereas the frequency of IFN-y or TNF-α-positive cells was high, but not different between the two groups. The decrease in peripheral MAIT cell frequency did not result from activation-induced cell death or exhaustion, since there was no difference in the mean fluorescence intensity of the survival marker Bcl2 and the T cell exhaustion markers TIM-3 and PD-1 in MAIT cells from the 2 groups. Rather, cirrhotic MAIT cells showed increased proliferation, as indicated by a higher frequency of Ki-67⁺MAIT cells compared to healthy donors. Finally, MAIT cells from healthy donors exposed to the plasma of cirrhotic patients showed an activated phenotype, characterized by an up-regulation of CD25 and CD69 expressions, whereas healthy MAIT cells exposed to healthy plasma had no effect. These data suggested that activation of blood MAIT cells may be due to soluble factor(s) present in the plasma of cirrhotic patients.

### MAIT cells accumulate along the fibrotic septa in livers from cirrhotic patients

We also studied the fate of MAIT cells in the cirrhotic liver, using isolated intrahepatic leukocytes from liver explants of patients undergoing transplantation. As expected ^{6,7}, the percentage of CD161⁺ Vα7.2⁺ MAIT cells was higher in the liver than in the blood in both control and cirrhotic livers (3.010%±0.38 in blood vs 12% in liver in healthy donors and 0.39%±0.11 vs 2.7% in liver in cirrhotic patients), but hepatic MAIT cell frequency was similar between cirrhotic patients and controls. However, immunohistochemistry of cirrhotic liver sections showed significant accumulation of CD3⁺Vα7.2⁺ positive cells along the fibrotic septa, with discrete or even no staining in the sinusoid, whereas control samples showed CD3⁺Vα7.2 immunoreactivity within the sinusoidal space. Cirrhotic liver MAIT cells showed an activated phenotype, characterized by higher frequencies of IL-17-producing MAIT cells, and a tendency to increase for Granzyme B, that did not reach significance. The number of IFN-y and TNF-α positive MAIT cells was high but not different between the two groups. In addition, MAIT cells expressed high levels of CD25 and CD69 in cirrhotic and healthy livers, but the frequencies of CD25⁺ and CD69⁺ MAIT cells were similar between the two groups. Finally, the frequencies of iNKT and γδT cells did not differ between the two groups.

Altogether, these data demonstrate that MAIT cell frequency is strongly decreased in the blood of cirrhotic patients, whereas there is a significant accumulation of MAIT cells along the fibrotic septa in the liver of these patients, in close contact with fibrogenic cells. Both in blood and liver of cirrhotic patients, MAIT cells show an activated phenotype as compared to that of healthy donors. We next investigated whether MAIT cells directly interact with hepatic fibrogenic cells and the functional consequences on their functions.

### Human MAIT cells enhance mitogenic and proinflammatory properties of human fibrogenic cells.

We first assessed in co-culture experiments whether MAIT cells directly stimulate fibrogenic cell proliferation. When human hepatic fibrogenic cells in their fully activated myofibroblastic phenotype ¹² were co-cultured with activated (anti-CD3/anti CD28/IL-7-exposed) MAIT cells, they showed enhanced Ki-67 staining or BrdU incorporation. In contrast, non-activated MAIT cells had a marginal effect on the proliferative capacity of hepatic myofibroblasts. Surprisingly, there was no stimulation of hepatic myofibroblast DNA synthesis by MAIT cells in transwell experiments. These data suggested that direct MAIT cell-hepatic myofibroblast contact rather than cytokine/chemokine production underlies MAIT cell-induced DNA synthesis of hepatic myofibroblasts. We hypothesized that TCR-dependent interaction via MR1 may be involved, since MR1 expression was revealed in hepatic myofibroblasts by immunostaining and FACS analysis, and strongly increased upon contact with MAIT cells. In addition, surface expression of MR1 on hepatic myofibroblasts was enhanced in response to Acetyl-6-formyl-pterin (Ac-6-FP), an MR1 ligand that stabilize MR1 at the plasma membrane¹⁴.**MAIT cell-induced proliferation of hepatic myofibroblasts was significantly blunted by a neutralizing anti MR1 antibody, whereas control isotype or anti-CD40 neutralizing antibody had no effect (****Figure 1A****).** Altogether, these data show that MAIT cells promotes accumulation of fibrogenic cells by stimulating their proliferation via an MR1-dependent pathway.

Hepatic myofibroblasts are also key contributors of the hepatic inflammatory response by producing chemokines and cytokines ^{1,2} and secrete proinflammatory mediators when stimulated by IL-17 or TNF-α ^{13,15}. Since MAIT cells are well characterized IL-17-and TNF-a-producing cells³, we investigated whether hepatic myofibroblasts may adopt a proinflammatory profile when exposed to activated MAIT cells. In co-culture experiments, activated MAIT cells enhanced the production of IL-6, TNF-a and IL-8 by hepatic myofibroblasts. Similar findings were observed in transwell experiments, as shown by FACS analysis and confirmed by ELISA, suggesting that mediators produced by MAIT cells enhance the proinflammatory properties of hepatic myofibroblasts. In keeping, the production of proinflammatory mediators in hepatic myofibroblasts was reduced when adding a TNF-a neutralizing antibody to the co-cultures, whereas the anti-IL17 antibody had no effect.

Collectively, these data reveal the profibrogenic and proinflammatory functions of MAIT cells, via distinct contact-mediated effect involving MR1, and cytokine-dependent pathways, respectively.

### Profibrogenic properties of MAIT cells in vivo

Since Vα7.2 + cells accumulated within the fibrotic septa in the cirrhotic liver and because MAIT cells enhance the mitogenic and proinflammatory properties of hepatic myofibroblasts, we investigated whether MAIT cells display fibrogenic properties in vivo, taking advantage of the availability of MAIT cell deficient mice (MR1-/-) and mice carrying a 10-fold increase in MAIT cell number (Vα19TCRTg). MR1-deficient mice showed no difference in liver injury, as shown by similar levels of serum transaminases. **However, MR1-deficient mice were resistant to fibrosis induced by CCl₄, as evidenced by decreased morphometry analysis of sirius red staining and lower number of fibrogenic cells (****Figure 1B****).** Conversely, MAIT cell-enriched mice exposed to CCl₄ displayed exacerbated fibrosis as compared to WT counterparts, as reflected by enhanced sirius red staining and increased number of α-smooth muscle actin-positive cells (α-SMA). Similar increases in Sirius red staining and accumulation of α-SMA positive cells were observed in Vα19TCRTg transgenic animals undergoing bile duct ligation as compared to WT counterparts. In contrast, we found no difference in the hepatic levels of TNF-α between either MR1-deficient or Vα19TCRTg as compared to their WT counterparts. Collectively, these data highlight the profibrogenic properties of MAIT cells in the liver.

### Discussion

Recent advances in the understanding of liver fibrosis pathogenesis have revealed that dysregulation of the immune system is a key factor leading to cirrhosis and liver failure, and suggested that manipulation of specific immune cell subsets may serve as the basis for antifibrotic strategies, in a pathological context where new therapies are urgently needed ^{2,3}. Combining human data in cirrhotic patients with cell culture experiments and in vivo models of fibrosis in MAIT cell-deficient or MAIT cell-enriched mice, the present study identifies activated MAIT cells as a major actor of the fibrogenic process.

Our data demonstrate that MAIT cell frequency is decreased in the blood of patients with chronic inflammatory liver diseases. However, MAIT cells from cirrhotic patients display an activated and proinflammatory profile, characterized by increased CD25 and CD69 expression and higher production of IL17 and granzyme B. These findings corroborate data obtained in patients with viral (HIV, HCV) or bacterial infections, or with inflammatory diseases, including inflammatory bowel disease, arthritic disease, systemic lupus erythromatosis, rheumatoid arthritis or type 2 diabetes (refs). The fate of circulating MAIT cells, and in particular whether they die or migrate to the target tissue has not been definitely addressed, due to the lack of appropriate tools to track these cells in vivo. However, our results indicate that the loss of circulating MAIT cells is unlikely due to cell death or exhaustion, as we found no difference in the number of BCl2, PDL3 or TIM-3-expressing MAIT cells between healthy and cirrhotic patients. More surprisingly, and although the number of MAIT cells is much higher in the liver than in the blood, we found no difference in MAIT cell number in the liver of cirrhotic individuals as compared to controls, but, as observed in the blood they displayed a proinflammatory phenotype, characterized by a higher frequency of IL17-positive cells. However, despite no change in hepatic MAIT cell frequency in the cirrhotic liver, immunhistochemistry experiments combining Vα7.2 and α-SMA immunostaining indicated that MAIT cells accumulate within the fibrotic septa, in close contact to hepatic fibrogenic cells. In contrast, MAIT cells were located in the sinusoidal space of control liver, in keeping with previous studies, but interestingly, moderate or no expression of MAIT cells was observed in the sinusoidal space of cirrhotic livers. However, additional studies are needed to determine whether local accumulation of activated MAIT cells in a fibrotic environment results from their redistribution within the liver and/or recruitment from the blood, therefore reflecting their decreased circulating frequency in cirrhotic patients. Yet, these data suggested that MAIT cells may interact with fibrogenic cells during chronic liver injury.

Combining in vivo studies and co-cultures experiments, our data identify MAIT cells as a novel pro-fibrogenic player. Indeed, MAIT cell-deficient or -overexpressing mice show miror decrease and increase in fibrosis, respectively, associated with a corresponding decrease and increase in the number of a-SMA positive fibrogenic cells. Interestingly, a major finding of our study is that enhanced proliferation of hepatic myofibroblasts by MAIT cells is a critical determinant of the profibrogenic function of MAIT cells. A key feature of the fibrogenic process is the high mitogenic capacity of hepatic fibrogenic cells that leads to their accumulation in the fibrotic septa during chronic liver injury. Proliferation of hepatic myofibroblasts is stimulated by a large variety of growth factors expressed during chronic liver injury, including PDGF (23); vasoconstrictors such as thrombin (24); the metalloproteinase MMP-2 (25); or adhesion molecules such as alphaVbeta3 integrins (26). However, co-culture experiments showed that the promitogenic properties of activated MAIT cells do not result from the release of mitogens for hepatic myofibroblasts but rather from direct cell-cell contact, since it was observed in co-cultures but not in transwell experiments. Because it has been reported that human hepatic fibrogenic cells express members of the human leucocyte antigen (HLA) family (HLA-I and HLA-II), lipid-presenting molecules (CD1b and CD1c) and factors involved in T cell activation (CD40 and CD80) and display features of antigen-presenting cells [22], we postulated that the mitogenic properties of MAIT cells may rely on TCR-dependent effects. **These data were corroborated by the identification of the non-classical MHC-related molecule MR1 at the cell membrane, both by FACS analysis and immunohistochemistry, and by the decrease in hepatic myofibroblast DNA synthesis upon incubation of co-cultures with a neutralizing MR1 antibody.** Strikingly however, co-culture experiments of hepatic myofibroblasts and in vitro activated MAIT cells showed that MAIT cells stimulate hepatic myofibroblast proliferation in the absence of MAIT ligand into the medium, suggesting that at least initially, MAIT cell-myofibroblast contact occurs via a TCR-dependent, antigen-independent pathway, as described for dendritic cell-T cell interaction. Whether during chronic liver injury, both antigen-dependent and antigen dependent pathways are initiated remains to be evaluated. Indeed, increase in gut permeability, intestinal bacteria overgrowth and dysbiosis are chacteristic features of patients with chronic liver diseases from various etiologies, allowing gut bacteria to flow to the liver. Similar findings have been reported in experimental models of chronic liver injury. These data suggest that in cirrhotic patients and in experimental models, bacterial-derived MAIT ligands generated from vitamin B2 metabolites and host-derived methylglyoxal could accumulate in the liver. Nevertheless, enhanced accumulation of hepatic myofibroblasts following MR1-dependent contact appears as a critical determinant of the profibrogenic functions of MAIT cells.

Another characteristic of hepatic fibrogenic cells is their pro-inflammatory properties. Our findings demonstrate that activated MAIT cells promotes a shift of hepatic myofibroblasts toward a proinflammatory phenotype that relies on the release of mediator produced by MAIT cells. Indeed, MAIT cell-induced production of IL8, IL6 and TNF-a by hepatic myofibroblasts is similarly observed in co-cultures and transwell experiments. Although TNF-a and IL17 were likely candiates release by MAIT cells, analysis of hepatic myofibroblast inflammatory profile showed a reduction in the production of IL8, IL6 and TNF-a by an anti TNF a neutralizing antibody whereas, surprisingly, IL17 had no or a marginal effects. These data indicated that MAIT cells stimulate the proinflammatory functions of hepatic myofibroblasts, via a TNF-a-dependent pathway. Nevertheless, it is likely that other mitogenic/pro-inflammatory mediators produced in low amounts by MAIT cells in culture experiments may contribute to MAIT cell-induced proinflammatory phenotype.

In conclusion, these data extend our knowledge on the general properties of MAIT cells. They also add to our understanding of the mechanisms underlying inflammation-driven fibrogenesis and unravel this non-conventional T cell subset as a promising target for antifibrogenic therapy.

### REFERENCES:

Throughout this application, various references describe the state of the art to which this invention pertains.
1. Mallat, A. & Lotersztajn, S. Cellular mechanisms of tissue fibrosis. 5. Novel insights into liver fibrosis. Am J Physiol Cell Physiol 305, C789-799 (2013).
2. Lotersztajn, S., Julien, B., Teixeira-Clerc, F., Grenard, P. & Mallat, A. Hepatic fibrosis : molecular mechanisms and drug targets. Ann Rev Pharmacol Toxicol 45, 605-628 (2005).
3. Franciszkiewicz, K., et al. MHC class I-related molecule, MR1, and mucosal-associated invariant T cells. Immunological reviews 272, 120-138 (2016).
4. Marra, F. & Lotersztajn, S. Pathophysiology of NASH: Perspectives for a Targeted Treatment. Current pharmaceutical design 19, 5250-5269 (2013).
5. Le Bourhis, L., et al. Antimicrobial activity of mucosal-associated invariant T cells. Nature immunology 11, 701-708 (2010).
6. Jeffery, H.C., et al. Biliary epithelium and liver B cells exposed to bacteria activate intrahepatic MAIT cells through MR1. J Hepatol 64, 1118-1127 (2016).
7. van Wilgenburg, B., et al. MAIT cells are activated during human viral infections. Nat Commun 7, 11653 (2016).
8. Kurioka, A., Walker, L.J., Klenerman, P. & Willberg, C.B. MAIT cells: new guardians of the liver. Clin Transl Immunology 5, e98 (2016).
9. Magalhaes, I., et al. Mucosal-associated invariant T cell alterations in obese and type 2 diabetic patients. The Journal of clinical investigation 125, 1752-1762 (2015).
10. Chiba, A., et al. Mucosal-associated invariant T cells promote inflammation and exacerbate disease in murine models of arthritis. Arthritis Rheum 64, 153-161 (2012).
11. Hiejima, E., et al. Reduced Numbers and Proapoptotic Features of Mucosal-associated Invariant T Cells as a Characteristic Finding in Patients with Inflammatory Bowel Disease. Inflammatory bowel diseases 21, 1529-1540 (2015).
12. Mallat, A., et al. Platelet-derived growth factor-BB and thrombin generate positive and negative signals for human hepatic stellate cell proliferation. Role of a prostaglandin/cyclic AMP pathway and cross-talk with endothelin receptors. The Journal of biological chemistry 273, 27300-27305 (1998).
13. Lemmers, A., et al. The interleukin-17 pathway is involved in human alcoholic liver disease. Hepatology (Baltimore, Md 49, 646-657 (2009).
14. Eckle, S.B., et al. A molecular basis underpinning the T cell receptor heterogeneity of mucosal-associated invariant T cells. The Journal of experimental medicine 211, 1585-1600 (2014).
15. Guillot, A., et al. Cannabinoid receptor 2 counteracts interleukin-17-induced immune and fibrogenic responses in mouse liver. Hepatology (Baltimore, Md 59, 296-306 (2014).
16. Bedossa, P. & Poynard, T. An algorithm for the grading of activity in chronic hepatitis C. The METAVIR Cooperative Study Group. Hepatology (Baltimore, Md 24, 289-293 (1996).
17. Weiss, E., et al. Type I interferon signaling in systemic immune cells from patients with alcoholic cirrhosis and its association with outcome. J Hepatol (2016).
18. Corbett, A.J., et al. T-cell activation by transitory neo-antigens derived from distinct microbial pathways. Nature 509, 361-365 (2014).
19. Tang, X.Z., et al. IL-7 licenses activation of human liver intrasinusoidal mucosal-associated invariant T cells. J Immunol 190, 3142-3152 (2013).
20. Mallat, A., et al. Growth inhibitory properties of endothelin-1 in activated human hepatic stellate cells: a cyclic adenosine monophosphate-mediated pathway. Inhibition of both extracellular signal-regulated kinase and c-Jun kinase and upregulation of endothelin B receptors. The Journal of clinical investigation 98, 2771-2778 (1996).
21. Teixeira-Clerc, F., et al. CB1 cannabinoid receptor antagonism: a novel strategy for the treatment of liver fibrosis. Nature medicine 12, 671-676 (2006).
22. Lodder, J., Chobert, M.N., Lotersztajn, S. & Teixeira-Clerc, F. Macrophage autophagy protects from liver fibrosis. Hepatology (Baltimore, Md 58, 283A (2013).

## Claims

1. An agent capable of inhibiting the activation of Mucosal-Associated Invariant T (MAIT) cells for use in a curative method of treating liver fibrosis in a patient in need thereof, wherein the agent is:
- An anti-MHC-related molecule 1 (MR1) neutralizing antibody or
- A small organic molecule selected from the group consisting of 6-formyl pterin and acetyl-6-formylpterin (Ac-6-FP).

2. The agent capable of inhibiting the activation of MAIT cells for use according to claim 1 wherein the liver fibrosis results from chronic alcohol consumption, overfeeding, insulin resistance, type 2 diabetes, non-alcoholic fatty liver disease, NASH, steatosis, idiopathic portal hypertension, autoimmune hepatitis, primary sclerosing cholangitis, or primary biliary cirrhosis.

3. The agent capable of inhibiting the activation of MAIT cells for use according to claim 1 wherein the liver fibrosis is associated with liver steatosis.

4. The agent capable of inhibiting the activation of MAIT cells for use according to claim 1 wherein the agent is an antibody that blocks the interaction between MR1 and Vα7.2-Jα33 receptors.

5. The agent capable of inhibiting the activation of MAIT cells for use according to claim 1 wherein the neutralizing antibody does not mediate antibody-dependent cell-mediated cytotoxicity and thus does not comprise an Fc portion that induces antibody dependent cellular cytotoxicity (ADCC).

## Patentansprüche

1. Wirkstoff, welcher die Aktivierung von Mucosa-assoziierten invarianten T- (MAIT-) Zellen hemmen kann, zur Verwendung in einem kurativen Verfahren zur Behandlung von Leberfibrose bei einem bedürftigen Patienten, wobei der Wirkstoff Folgendes ist:
- ein Anti-MHC-verwandtes Molekül 1 (MR1) neutralisierender Antikörper oder
- ein kleines organisches Molekül, ausgewählt aus der Gruppe bestehend aus 6-Formylpterin und Acetyl-6-Formylpterin (Ac-6-FP).

2. Wirkstoff, welcher die Aktivierung von MAIT-Zellen hemmen kann, zur Verwendung nach Anspruch 1, wobei die Leberfibrose aus chronischem Alkoholkonsum, Überernährung, Insulinresistenz, Typ-2-Diabetes, nichtalkoholischer Fettlebererkrankung, NASH, Steatose, idiopathischer portaler Hypertension, Autoimmunhepatitis, primärer sklerosierender Cholangitis oder primärer biliärer Zirrhose resultiert.

3. Wirkstoff, welcher die Aktivierung von MAIT-Zellen hemmen kann, zur Verwendung nach Anspruch 1, wobei die Leberfibrose mit Lebersteatose assoziiert ist.

4. Wirkstoff, welcher die Aktivierung von MAIT-Zellen hemmen kann, zur Verwendung nach Anspruch 1, wobei es sich bei dem Wirkstoff um einen Antikörper handelt, welcher die Interaktion zwischen MR1- und Vα7.2-Jα33-Rezeptoren blockiert.

5. Wirkstoff, welcher die Aktivierung von MAIT-Zellen hemmen kann, zur Verwendung nach Anspruch 1, wobei der neutralisierende Antikörper keine antikörperabhängige zellvermittelte Zytotoxizität vermittelt und daher keinen Fc-Teil umfasst, welcher eine antikörperabhängige zelluläre Zytotoxizität (ADCC) induziert.

## Revendications

1. Agent capable d'inhiber l'activation des cellules T invariantes associées aux muqueuses (MAIT) pour une utilisation dans un procédé curatif de traitement de la fibrose hépatique chez un patient qui en a besoin, dans lequel l'agent est :
- un anticorps neutralisant dirigé contre la molécule 1 associée à l'anti-MHC (MR1) ou
- une petite molécule organique sélectionnée dans le groupe consistant en la 6-formylptérine et l'acétyl-6-formylptérine (Ac-6-FP).

2. Agent capable d'inhiber l'activation des cellules MAIT pour une utilisation selon la revendication 1, dans lequel la fibrose hépatique résulte d'une consommation chronique d'alcool, d'une suralimentation, d'une résistance à l'insuline, d'un diabète de type 2, d'une stéatose hépatique non alcoolique, d'une NASH, d'une stéatose, d'une hypertension portale idiopathique, d'une hépatite auto-immune, d'une cholangite sclérosante primitive ou d'une cirrhose biliaire primitive.

3. Agent capable d'inhiber l'activation des cellules MAIT pour utilisation selon la revendication 1, dans lequel la fibrose hépatique est associée à une stéatose hépatique.

4. Agent capable d'inhiber l'activation des cellules MAIT pour utilisation selon la revendication 1, dans lequel l'agent est un anticorps qui bloque l'interaction entre les récepteurs MR1 et Vα7.2-Jα33.

5. Agent capable d'inhiber l'activation des cellules MAIT pour utilisation selon la revendication 1, dans lequel l'anticorps neutralisant ne médie pas la cytotoxicité cellulaire dépendante des anticorps et ne comprend donc pas de portion Fc qui induit la cytotoxicité cellulaire dépendante des anticorps (ADCC).
